# EUROPEAN PATENT APPLICATION

(11) **EP 3 553 053 A1**
(43) Date of publication of application: **16.10.2019**
(21) Application number: 18167065.4
(22) Date of filing: 12.04.2018
(51) Int. Cl.: C07D 277/26, A61P 25/00, A61K 31/426

(54) **(S)-ENANTIOMERIC FORM OF A HETEROCYCLIC COMPOUND HAVING MOTIVATION IMPROVING AND/OR REFERENCE MEMORY ENHANCING ACTIVITY**

(71) Applicant: Red Bull GmbH, 5330 Fuschl am See (AT)
(72) Inventor: Lubec, Gert, 1220 Wien (AT)
(74) Representative: Metten, Karl-Heinz

(57) **Abstract**

The present invention relates to the (*S*)-enantiomer of the chemical compound having the general formula (I): 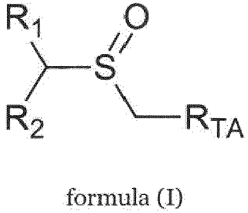 wherein R₁ and R₂ are, equal or independently, aryl, heteroaryl, fused aryl, fused heteroaryl, mono or multi-substituted aryl, mono or multi-substituted heteroaryl, mono or multi-substituted fused aryl, mono or multi-substituted fused heteroaryl; and R_{TA} is a 2-1,3-, or 4-1,3- or 5-1,3-thiazole ring with the general Formula (IIa), which can also be substituted.

The invention also relates to said compound according to formula (I) for use in improving motivation and/or cognitive functions and/or for use in improving the reference memory in human individuals.

## Description

The present invention relates to enantiomeric compounds that can be used to improve motivation and cognitive functions and in particular reference memory in human individuals, in particular in aging individuals. In particular, the present invention refers to new compounds which can improve motivation, learning capabilities and /or reference memory performance and which can be used to treat age-dependent decline of cognitive functions as well as motivation.

While working memory can be considered to be a specific form of short-term memory that refers to the ability to retain information within a single trial, i.e. to actively hold multiple pieces of transitory information in the mind where they can be manipulated, the technical term "reference memory" refers to the long-term storage of information that remains constant over time and that is gradually acquired over many training sessions. That is, in contrast to working memory, reference memory appears to be more comparable to long-term memory because it refers to the ability to store information about a specific fixed situation. In general, reference memory involves storing information that remains constant over time. According to Fauci et al. (in "Harrison's Principles of Internal Medicine", volume 1, pp. 142 to 150, 1988) reference memory refers to information gained from previous experience, either recent or remote, i.e. reference memory refers to knowledge for a certain situation that remains constant over time. As stated by A.N. Guitar in "The Interaction Between Spatial Working and Reference Memory in Rats on a Radial Maze" (Paper 4; 2014; http://ir.lib.uwo.ca/psychd_uht) working memory and reference memory could be defined as independent systems both cognitively and physically.

Working memory is the system that actively holds multiple pieces of transitory information in the mind, where they can be manipulated in a way that makes them useful for goal directed behaviour. This involves execution of verbal and nonverbal tasks, such as reasoning and comprehension and makes them available for further information-processing (Becker JT et al. 1999 Brain Cogn 41 (1): 1-8). Thus it is considered that working memory includes subsystems that store and manipulate visual images or verbal information, resp., and also a central executive that coordinates these subsystems. It has been proposed to include visual representation of the possible moves, and awareness of the flow of information into and out of memory, all stored for a limited amount of time (Schacter, Daniel (2009, 2011), Psychology Second Edition, United States of America, Worth Publishers, p. 227). The concept of a working memory is generally based on the Baddeley's model as proposed in 1974 and supplemented in 2000 (Baddeley, Alan D.; Hitch, Graham (1974). "Working Memory", in Gor-don H. Bower: "The psychology of learning and motivation 2", Academic Press, pages 47 to 89; Baddeley A. November 2000, "The episodic buffer: a new component of working memory?", Trends Cogn. Sci. (Regul. Ed.) 4 (11), pages 417 to 423). Working memory tasks are considered to require monitoring (i.e., manipulation of information or behaviors) as part of completing goal-directed actions in the setting of interfering processes and distractions. The cognitive processes needed to achieve this include the executive and attention control of short-term memory, which permit interim integration, processing, disposal, and retrieval of information. These processes have been found to be sensitive to age.

It is also well accepted that short-term memory is the capacity for holding a small amount of information in mind in an active, readily available state for a short period of time. The duration of short-term memory is believed to be in the order of seconds. A commonly cited capacity is 7 ± 2 elements which can be simultaneously kept in the short-term memory (Miller G., March 1956, "The magical number seven plus or minus two: some limits on our capacity for processing information", Psychological Review 63 (2): 81-979). In contrast, long-term memory can hold an indefinite amount of information.

It is recognized that the enantiomers of a chiral drug may possess pharmacokinetic and pharmacodynamic properties different from their racemates. There is thus a risk for misinterpretation of drug disposition and plasma drug concentration-effect data generated for a racemic drug using a non-stereoselective assay. The data collected for the racemic form of a drug is frequently both quantitatively and qualitatively inaccurate with respect to the individual enantiomers. For example, the clearance of the unresolved drug may indicate concentration- and time-dependence even though this pharmacokinetic process is concentration-and time-independent for each of the enantiomers.

C. J. Loland et al., Biol. Psychiatry, 2012 September 1, 72 (5), pages 405 to 413 ("R-Modafinil (Armodafinil): A unique dopamine up-take inhibitor and potential medication for psychostimulant abuse"), found out that R-modafinil displays an in vitro profile different from that of cocaine. It was, however, concluded that further trials with R-modafinil as a substitute therapy are warranted.

In WO 03/059873 A1 pharmaceutical compounds are disclosed comprising both a thiazole group and a quaternary carbon comprising three phenyl groups next to a sulfoxide moiety. These compounds shall furnish a medicament which regulates calcium-activated potassium channels and which acts as modulator of SKca and/or IKca channels. According to this document the proposed pharmaceutical compounds shall be suited for the treatment of diseases or conditions like respiratory diseases such as asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary heart disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type 11, hyperinsulinemia, premature labour, baldness, cancer, and immune suppression in particular in for reducing or inhibiting undesired immune-regulatory actions, Addison's disease, alopecia areata, Ankylosing spondylitis, haemolytic anemia (anemia haemolytica), pernicious anemia (anemia perniciosa), aphthae, aphthous stomatitis, arthritis, arteriosclerotic disorders, osteoarthritis, rheumatoid arthritis, aspermiogenese, asthma bronchiale, auto-immune asthma, auto-immune hemolysis, Bechet's disease, Boeck's disease, inflammatory bowel disease, Burkitt's lymphom, Chron's disease, chorioiditis, colitis ulcerosa, Coeliac disease, cryoglobulinemia, dermatitis herpetiformis, dermatomyositis, insulin-dependent type I diabetes, juvenile diabetes, idiopathic diabetes insipidus, insulin-dependent diabetes mellisis, auto- immune demyelinating diseases, Dupuytren's contracture, encephalomyelitis, encephalomyelitis allergic, endophthalmia phacoanaphylactica, enteritis allergic, auto-immune enteropathy syndrome, erythema nodosum leprosum, idiopathic facial paralysis, chronic fatigue syndrome, febris rheumatica, glomerulo nephritis, Goodpasture's syndrome, Graves'disease, Hamman-Rich's disease, Hashimoto's disease, Hashimoto's thyroiditis, sudden hearing loss, sensoneural hearing loss, hepatitis chronica, Hodgkin's disease, haemoglobinuria paroxysmatica, hypogonadism, ileitis regionalis, iritis, leucopenia, leucemia, lupus erythematosus disseminatus, systemic lupus erythematosus, cutaneous lupus erythematosus, lymphogranuloma malignum, mononucleosis infectiosa, myasthenia gravis, traverse myelitis, primary idiopathic myxedema, nephrosis, ophthalmia symphatica, orchitis granulomatosa, pancreatitis, pemphigus, pemphigus vulgaris, polyarteritis nodosa, polyarthritis chronica primaria, polymyositis, polyradiculitis acuta, psoreasis, purpura, pyoderma gangrenosum, Quervain's thyreoiditis, Reiter's syndrome, sarcoidosis, ataxic sclerosis, progressive systemic sclerosis, scleritis, sclerodermia, multiple sclerosis, sclerosis disseminata, acquired spenic atrophy, infertility due to antispermatozoan antobodies, thrombocytopenia, idiopathic thrombocytopenia purpura, thymoma, acute anterior uveitis, vitiligo, AIDS, HIV, SCID and Epstein Barr virus associated diseases such as Sjorgren's syndrome, virus (AIDS or EBV) associated B cell lymphom, parasitic diseases such as Le-sihmania, and immunosuppressed disease states such as viral infections following allograft transplantations, graft vs. Host syndrome, transplant rejection, or AIDS, cancers, chronic active hepatitis diabetes, toxic chock syndrome, food poisoning, and transplant rejection.

US 2002/0183334 is about substituted thioacetamides. The rather broad set of compounds disclosed in US 2002/0183334 shall be suited for the treatment of sleepiness, tiredness, Parkinson's disease, cerebral ischemia, stroke, sleep apneas, eating disorders, attention deficit hyperactivity disorder, cognitive dysfunction, disorders associated with hypofunctionality of the cerebral cortex, depression, schizophrenia, and chronic fatigue syndrome, and also for the promotion of wakefulness, stimulation of appetite, or stimulation of weight gain.

In WO 03/066035 a broad range of thioether sulfonamides is disclosed which shall be suited for the treatment of diabetes mellitus.

US 2005/0234040 refers to tricyclic aromatic and bis-phenyl sulfinyl derivatives. The compounds specified in this document shall furnish medicaments suited for the treatment of excessive sleepiness, promotion and/or improvement of wakefulness, preferably improvement of wakefulness in patients with excessive sleepiness associated with narcolepsy, sleep apnea, preferably obstructive sleep apnea/hypopnea, and shift work disorder), Parkinson's disease, Alzheimer's disease, cerebral ischemia, stroke, eating disorders, attention deficit disorder ("ADD"), attention deficit hyperactivity disorder ("ADHD"), depression, schizophrenia, fatigue, preferably fatigue associated with cancer or neurological diseases, such as multiple sclerosis and chronic fatigue syndrome, stimulation of appetite and weight gain and improvement of cognitive dysfunction.

From EP 2 292 213 A1 a pharmaceutical composition consisting of a specific polymorphic form of R-(-)-modafinil and one or more pharmaceutically-acceptable carriers, diluents or excipients can be derived by use of which subjects with narcolepsy can be treated ensuring a longer lasting plasma concentration and higher overall exposure at high doses when compared to equivalent amounts of the racemic form of modafinil.

Although numerous attempts have been made it would be desirable to provide a way which would allow to efficiently and effectively cope with known memory deficits. Compounds and methods which exhibit a further improvement of memory functions are still sought. It is therefore an object of the present invention to provide agents that increase cognitive performance, in particular in both impaired as well as in healthy subjects. It is another object of the present invention to treat age-dependent decline of cognitive functions, and to provide an effective agent which can be used for the treatment of age-dependent decline and/or of disorders that in turn require improvement of memory functions. It is a further object of the present invention to improve motivation in healthy and disabled persons.

Therefore, the present invention provides the (*S*)-enantiomer of the chemical compound having the general formula (I): 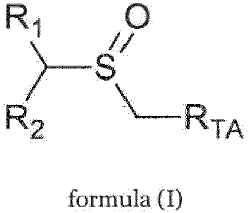 wherein R₁ and R₂ are, equal or independently, aryl, heteroaryl, fused aryl, fused heteroaryl, mono or multi-substituted aryl, mono or multi-substituted heteroaryl, mono or multi-substituted fused aryl, mono or multi-substituted fused heteroaryl;
R_{TA} is a 2-1,3-, or 4-1,3- or 5-1,3-thiazole ring with the general Formula (IIa), 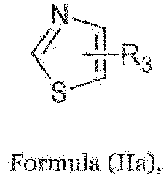 R₃ is present 1 or 2 times, equal or independently, wherein R₃ is selected from the group consisting of hydrogen, alkyl, cycloalkyl, heterocycloalkyl, alkylamino, in particular dialkylamino, arylamino, in particular diarylamino, hydroxyalkylamino, alkoxy, arylalkyl, hydroxyalkyl, thioalkyl, haloalkyl, haloaryl, haloarylalkyl, haloalkoxy, mono or multi-substituted alkyl, mono or multi-substituted cycloalkyl, mono or multi-substituted heterocycloalkyl, mono or multi-substituted alkylamino, in particular dialkylamino, mono or multi-substituted arylamino, in particular diarylamino, mono or multi-substituted hydroxyalkylamino, mono or multi-substituted alkoxy, mono or multi-substituted arylalkyl, mono or multi-substituted hydroxyalkyl, mono or multi-substituted thioalkyl, mono or multi-substituted haloalkyl, mono or multi-substituted haloaryl, mono or multi-substituted haloarylalkyl, mono or multi-substituted haloalkoxy and carboxylate ester, wherein substituted in the meaning of the present invention means substituted with a residue selected from the group consisting of alkyl, cykloalkyl, heterocycloalkyl, hydroxyalkyl, alkylthio, ether, hydroxyl, fluoride, chloride, bromide and iodide.

It has been found that those compounds according to formula (I) are preferred in which R₃ is hydrogen or alkyl, in particular methyl, or hydroxyalkyl, in particular hydroxymethyl. Those compounds according to formula (I) are also preferred in which R₃ is alkyl substituted with at least one residue selected from the group consisting of heterocycloalkyl, carboxylic acid, amide and ester. Even more preferred in this regard are R₃ residues in which alkyl is methyl substituted with heterocycloalkyl, preferably heterocycloalkyl containing at least two heteroatoms. That is, in this embodiment alkyl respresents an alkylene bridging group, in particular a methylene bridging group, between the heterocycle according to formula (IIa) and the heterocycloalkyl group. Heterocycloalkyl is preferably selected from the group consisting of morpholino, piperazino and methylpiperazino.

Aryl, i.e.an aryl group in the meaning of the present invention preferably denotes phenyl, and substituted aryl preferably denotes mono or multi-substituted phenyl. Exemplary substituted phenyl include for example -o-C₆H₄-R', -m-C₆H₄-R' and -p-C₆H₄-R', wherein R' is an alkyl as defined herein or as defined for residues R₃, R₄, R₅, R₆ and R₇. Fused aryl in the meaning of the present invention preferably denotes an aromatic ring being fused with at least one aromatic ring system, the fused aryl group in particular being made of five to 15 carbon atoms, e.g. naphthyl and anthracenyl. These fused aryl may also be mono or multi-substituted, e.g. 1-naphthyl, 2-naphthyl, 1-anthracenyl or 2-anthracenyl, as defined herein.

Heteroaryl, i.e. a heteroaryl group in the meaning of the present invention preferably denotes a 3 to 8-membered heterocyclic aromatic group which contains at least one heteroatom selected from the group consisting of O, N, and S. Heteroaryl in the meaning of the present invention also includes a heteroaryl ring being fused to another aromatic ring. Both heteroaryl and fused heteroaryl group can also be mono or multi-substituted as defined herein.

Alkyl, i.e. an alkyl group in the meaning of the present invention preferably comprises an alkyl, alkenyl and alkynyl residue, in particular a C₁-C₆-alkyl, C₂-C₆-alkenyl and C₂-C₆-alkynyl residue. Suitable residues are selected from the group consisting of -CH₃, -C₂H₅,-CH=CH2, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇,
-C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅,-CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H_{5,} -CH₂-C(CH₃)=C(CH₃)₂, -C₂H₄-CH=C(CH₃)₂, -C₄H₈-C≡CH, -C≡C-C₄H₉, -C₃H₆-C≡C-CH₃,
-CH₂-C≡C-C₃H₇, and -C₂H₄-C≡C-C₂H₅;
wherein R' can be defined as residue R₃ as outlined above for formula (I). R' as referred to above preferably is hydrogen, methyl, ethyl, n-propyl, i-propyl, n-butyl, tert-butyl, sec-butyl or isobutyl, in particular hydrogen, methyl or i-propyl.

The alkyl group according to one preferred embodiment of formula (I), in particular for residue R₃, is methyl, ethyl, propyl, isopropyl, n-butyl, tert-butyl, sec-butyl, and isobutyl, preferably methyl or isopropyl, and more preferably alkyl is selected from the group consisting of methyl, ethyl, iso-propyl, and tert-butyl.

Arylalkyl, i.e. an arylalkyl group in the meaning of the present invention preferably denotes a linear or branched C₁-C₆-alkyl substituted with at least one aryl group as defined above, preferably benzyl or phenylethyl. Suitable mono or multi-substituted arylalkyl comprise for example 4-hydroxybenzyl, 3-fluorobenzyl or 2-fluorophenylethyl.

Cycloalkyl, i.e. a cycloalkyl group in the meaning of the present invention preferably denotes a non-aromatic ring system containing three to eight carbon atoms, preferably four to eight carbon atoms. With mono or multi-substituted cycloalkyl one or more of the carbon atoms in the ring may be substituted by a group R' as defined above, preferably methyl, ethyl, n-propyl, i-propyl, n-, i- or t-butyl. Suitable C₃-C₈-cycloalkyl groups can be selected from the group consisting of -cyclo-C₃H₅, -cyclo-C₄H₇, -cyclo-C₅H₉, -cyclo-C₆H₁₁, -cyclo-C₇H₁₃, and -cyclo-C₈H₁₅.

Heterocycloalkyl, i.e. heterocycloalkyl group in the meaning of the present invention preferably denotes a non-aromatic carbon ring system in which one or more ring carbon atoms have been replaced by a heteroatom functional group such as O, S, SO, SO₂, N, or NR'2, wherein R' is defined as outlined above. Preferred heterocycloalkyl groups can be selected from the group consisting of morpholine-4-yl, piperazinyl, and 1-alkylpiperazine-4-yl.

Hydroxyalkyl, i.e. a hydroxyalkyl group in the meaning of the present invention preferably denotes a hydroxyalkyl group in which the alkyl group is defined as outlined above, preferably methyl, ethyl, n-propyl, i-propyl, n-, i- or t-butyl. Suitable hydroxyalkyl groups are selected from the groups consisting of a hydroxymethyl, hydroxyethyl, hydroxypropyl, and hydroxy-iso-propyl group. The hydroxyalkyl group according to a preferred embodiment of formula (I) is hydroxymethyl. Once bonded the alkyl or methyl group may also be denoted as alkylene or methylene group, respectively.

Alkylthio, i.e. an alkylthio group in the meaning of the present invention preferably denotes an alkylthio group in which the alkyl group is defined as outlined above, preferably for R₃, in particular thiomethyl.

Haloalkyl, i.e. a haloalkyl group in the meaning of the present invention preferably denotes a haloalkyl group in which the alkyl group is defined as outlined above, in particular for R₃, and wherein said alkyl is substituted by one or more halogen atoms, preferably substituted by one to five halogen atoms, in particular by fluor or chlorine atoms. In a preferred embodiment the haloalkyl group is selected from the group consisting of -C(R¹⁰)₃, -CR¹⁰(R^{10'})₂, -CR¹⁰(R^{10'})R^{10"}, -C₂(R¹⁰)₅, -CH₂-C(R¹⁰)₃, -C(R^{10'})₂-CH(R^{10'})₂, -CH₂-CR¹⁰(R^{10'})2, -CH₂-CR¹⁰(R^{10'})R^{10"}, -C₃(R¹⁰)₇, or -C₂H₄-C(R¹⁰)₃, wherein R¹⁰, R^{10'}, R^{10"} represent F, Cl, Br or I, preferably F.

Haloaryl, i.e. a haloaryl group in the meaning of the present invention preferably denotes a haloaryl group in which at least one aromatic carbon atom is substituted by one or more halogen atoms, preferably substituted by one to five halogen atoms. Preferably, halogen atoms are selected from the group consisting of fluorine, chlorine and bromine atoms, and preferably fluorine.

Haloarylalkyl, i.e. a haloarylalkyl group in the meaning of the present invention preferably denotes a haloarylalkyl group in which the arylalkyl group is defined as outlined above and wherein at least one aromatic carbon atom of said aryl group is substituted by one or more halogen atoms, preferably substituted by one to five halogen atoms. Preferably, halogen atoms are selected from the group consisting of fluorine, chlorine, bromine and iodine atoms, more preferably from fluorine and chlorine atoms.

Haloalkoxy, i.e. a haloalkoxy group in the meaning of the present invention preferably denotes a haloalkoxy group in which the alkoxy group is defined as outlined above and wherein said alkoxy is substituted by one or more halogen atoms, preferably substituted by one to five halogen atoms, in particular by fluorine, chlorine, bromine and/or iodine atoms, more preferably fluorine and/or chlorine atoms. Preferably, the haloalkoxy group is selected from the group consisting of -OC(R¹⁰)₃, -OCR¹⁰(R^{10'})₂, -OCR₁₀(R^{10'})R^{10"}, -OC₂(R¹⁰)₅, -OCH₂-C(R¹⁰)₃, -OCH₂-CR¹⁰(R^{10'})₂, -OCH₂₋CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F.

Alkylamino, i.e. an alkylamino group in the meaning of the present invention preferably denotes an alkylamino group in which the alkyl group is defined as outlined above, in particular for R₃. The alkylamino group in the meaning of the present invention preferably denotes a (alkyl)₂-N-group, i.e. a dialkylamino group, or a alkyl-NH- group. The dialkylamino group is preferred. Alkyl is preferably defined as outlined above.

Arylamino, i.e. a diarylamino group in the meaning of the present invention preferably denotes an arylamino group in which the aryl group is defined as outlined above. The arylamino group denotes a (aryl)₂-N-group, i.e. a diarylamino group, or a aryl-NH- group. The diarylamino group is preferred, in particular the diphenylamino group. Aryl is preferably defined as outlined above.

Hydroxyalkylamino, i.e. an hydroxyalkylamino group in the meaning of the present invention preferably denotes an hydroxyalkylamino group in which the alkyl group is defined as outlined above, in particular for R₃, and wherein the hydroxyalkylamino group denotes a (HO-alkyl)₂-N-group or a HO-alkyl-NH- group, or a (HO-alkyl)-N-(alkyl)- group. Alkyl is preferably defined as outlined above.

Those compounds according to formula (I) are particularly preferred in which at least one of R₁ and R₂ is aryl or substituted aryl, preferably phenyl or substituted phenyl, e.g. tolyl.

According to another embodiment of the compound of formula (I) R₁ is aryl or substituted aryl, preferably phenyl or substituted phenyl, e.g. tolyl, and R₂ is heteroaryl or substituted heteroaryl.

According to an embodiment of the compound of formula (I) having a 1,3,4-oxadiazole ring with the formula (IIc) R₃ is hydrogen and R₁ and R₂ are aryl or substituted aryl, preferably phenyl.

It has been found that most promising results can be obtained when in formula (I) both R₁ and R₂ are phenyl or substituted phenyl, preferably phenyl. Moreover, those (S) enantiomers of the compounds according to formula (I) furnish particularly improved results in terms of motivation and/or cognitive functions such as learning capabilities, reference memory, in particular motivation and reference memory, in which R_{TA} is a thiazole group, preferably a 2-thiazole group, especially 2-thiazole, or a 4-thiazole group, in particular 2-methyl-4-thiazole. In another preferred embodiment R_{TA} is a thiadiazole group, preferably a 1,3,4-thiadiazole-5-oxyalkyl group, especially 1,3,4-thiadiazole-5-oxymethyl. And, in another preferred embodiment of the compound according to formula (I) R_{TA} is a 1,3,4-oxadiazole group, preferably 1,3,4-oxadiazole.

Particularly preferred S enantiomers of the compounds according to formula (I) in terms of improving motivation and/or cognitive functions such as learning capabilities, in particular motivation and reference memory, more preferably motivation and reference memory, are those selected from the group consisting of the S enantiomer of
2-(diphenylmethanesulfmyl methyl)-1,3-thiazole,
4-(diphenylmethanesulfinylmethyl)-1,3-thiazole,
2-(diphenylmethanesulnnylmethyl)-4-methyl-1,3-thiazole,
5-(diphenylmethanesulfinylmethyl)-1,3-thiazole and
4-(diphenylmethanesulfinylmethyl)-2-methyl-1,3-thiazole,
5-(diphenylmethanesulfinylmethyl)-1,3-thiazole,
5-(diphenylmethanesulfinylmethyl)-2-chloro-1,3-thiazole,
5-(diphenylmethanesulfinylmethyl)-4-methyl-1,3-thiazole,
5-(diphenylmethanesulfinylmethyl)-4-chloro-1,3-thiazole,
5-(diphenylmethanesulfinylmethyl)-2-methyl-4-methyl-1,3-thiazole,
5-(diphenylmethanesulfinylmethyl)-2-methyl-4-chloro-1,3-thiazole,
5-(diphenylmethanesulfinylmethyl)-2-chloro-4-methyl-1,3-thiazole,
5-(diphenylmethanesulfinylmethyl)-2-cyclopropyl-1,3-thiazole,
4-(diphenylmethanesulfinylmethyl)-2-oxirane-1,3-thiazole,
4-(diphenylmethanesulfinylmethyl)-2-cyclopropyl-5-methyl-1,3-thiazole,
4-(diphenylmethanesulfinylmethyl)-2-cyclopropyl-5-chloro-1,3-thiazole,
4-(diphenylmethanesulfinylmethyl)-2-cyclopropyl-1,3-thiazole.

Particularly preferred in this context, particularly with regard to motivation and/or reference memory improvement, more in particular with regard to motivation and reference memory improvement, is the (*S*)-enantiomer of 4-(diphenylmethanesulfinylmethyl)-2-methyl-1,3-thiazole and/or 5-(diphenylmethanesulfinylmethyl)-1,3-thiazole, more in particular of 5-(diphenylmethanesulfinylmethyl)-1,3-thiazole.

Particularly preferred are the S enantiomers of the chemical compounds having the general formula (I), wherein R₃ is present on the thiazole ring R_{TA} according to Formula (IIa) 2 times and independently selected from the group consisting of R₄, R₅, R₆ and R₇, wherein R₁ and R₂ are, equal or independently, aryl or substituted aryl, preferably both are phenyl, wherein R_{TA} is a 4-1,3-thiazole ring with the general Formula (IIIa) 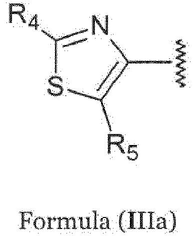 wherein R₄ is selected from the group consisting of oxirane, cyclopropyl, cyclobutyl, cyclopentyl and cyclohexyl, preferably cyclopropyl, and wherein R₅ is selected from the group consisting of hydrogen, methyl, fluoro, chloro, bromo, iodo, n-propyl, iso-propyl, preferably hydrogen, or wherein R_{TA} is a 5-1,3-thiazole ring with the general Formula (IIIb) 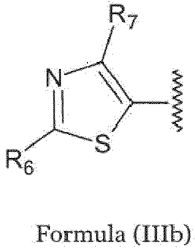 wherein R₆ is selected from the group consisting of hydrogen, methyl, n-propyl, iso-propyl, cyclopropyl, fluoro, chloro, bromo, iodo, cyclobutyl, cyclopentyl and cyclohexyl, preferably hydrogen, and wherein R₇ is selected from the group consisting of hydrogen, methyl, n-propyl, iso-propyl, fluoro, chloro, bromo, iodo, cyclopropyl, cyclobutyl, cyclopentyl and cyclohexyl, preferably hydrogen.

The invention further relates to the S enantiomers of the chemical compound having the general formula (I): 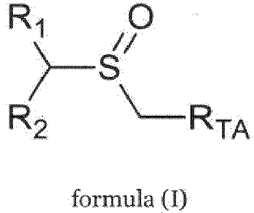 wherein R₁ and R₂ are, equal or independently, aryl or substituted aryl, preferably both are phenyl, wherein R_{TA} is a 4-1,3-thiazole ring with the general Formula (IIIa) 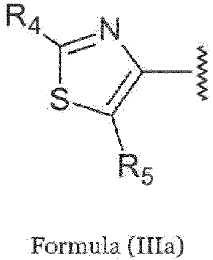 wherein R₄ is selected from the group consisting of oxirane, cyclopropyl, cyclobutyl, cyclopentyl and cyclohexyl, preferably cyclopropyl, and wherein R₅ is selected from the group consisting of hydrogen, methyl, n-propyl, iso-propyl, fluoro, chloro, bromo and iodo, preferably hydrogen, or wherein R_{TA} is a 5-1,3-thiazole ring with the general Formula (IIIb) 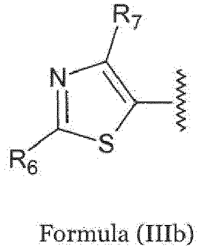 wherein R₆ is selected from the group consisting of hydrogen, methyl, n-propyl, iso-propyl, fluoro, chloro, bromo, iodo, cyclopropyl, cyclobutyl, cyclopentyl and cyclohexyl, preferably hydrogen, and wherein R₇ is selected from the group consisting of hydrogen, methyl, n-propyl, iso-propyl, fluoro, chloro, bromo, iodo, cyclopropyl, cyclobutyl, cyclopentyl and cyclohexyl, preferably hydrogen.

Those S enantiomers of the compounds according to formula (I) are particularly preferred in which at least one of R₁ and R₂ is aryl or substituted aryl, preferably phenyl or substituted phenyl, e.g. tolyl. It has been found that most promising results can be obtained when in formula (I) both R₁ and R₂ are phenyl or substituted phenyl, preferably phenyl.

In a preferred embodiment of the compound according to formula (I) substituted in the meaning of the present invention preferably means substituted with a residue selected from the group consisting of alkyl, cykloalkyl, heterocycloalkyl, hydroxyalkyl, alkylthio, ether, hydroxyl, fluoride, chloride, bromide and iodide. The alkyl group according to one preferred embodiment of formula (I) is methyl, ethyl, propyl, isopropyl, n-butyl, tert-butyl, sec-butyl, and isobutyl, preferably methyl or isopropyl, and more preferably alkyl is selected from the group consisting of methyl, ethyl, iso-propyl, and tert-butyl.

Those compounds according to formula (I) having residue (IIIa) are particularly preferred in which R₄ is cyclopropyl and wherein R₅ is hydrogen.

Moreover, those compounds according to formula (I) having residue (IIIb) are particularly preferred in which both R₆ and R₇ are hydrogen.

And, those S enantiomers of the compounds according to formula (I) furnish particularly improved results in terms of motivation and/or cognitive functions such as learning capabilities, reference memory, in particular motivation and reference memory, which are selected from the group consisting of the S enantiomer compound of
5-(diphenylmethanesulfinylmethyl)-1,3-thiazole,
5-(diphenylmethanesulfinylmethyl)-2-chloro-1,3-thiazole,
5-(diphenylmethanesulfinylmethyl)-2-methyl-1,3-thiazole,
5-(diphenylmethanesulfinylmethyl)-4-methyl-1,3-thiazole,
5-(diphenylmethanesulfinylmethyl)-4-chloro-1,3-thiazole,
5-(diphenylmethanesulfinylmethyl)-2-methyl-4-methyl-1,3-thiazole,
5-(diphenylmethanesulfinylmethyl)-2-methyl-4-chloro-1,3-thiazole,
5-(diphenylmethanesulfinylmethyl)-2-chloro-4-methyl-1,3-thiazole,
5-(diphenylmethanesulfinylmethyl)-2-cyclopropyl-1,3-thiazole,
4-(diphenylmethanesulfinylmethyl)-2-cyclopropyl-5-methyl-1,3-thiazole,
4-(diphenylmethanesulfinylmethyl)-2-cyclopropyl-5-chloro-1,3-thiazole,
4-(diphenylmethanesulfinylmethyl)-2-oxirane-1,3-thiazole, and
4-(diphenylmethanesulfinylmethyl)-2-cyclopropyl-1,3-thiazole.

Preferred compounds according to formula (I) in terms of improving motivation and/or cognitive functions, such as learning capabilitiers, in particular motivation and reference memory, are those selected from the group consisting of (S)-4-(diphenylmethanesulfinylmethyl)-2-cyclopropyl-1,3-thiazole (compound (I) having residue (IIIa)) and (*S*)-5-(diphenylmethanesulfinylmethyl)-1,3-thiazole (also referred to as (*S*)-5-((benzhydrylsulfinyl)methyl)-1,3-thiazole) (compound (I) having residue (IIIb)). Particularly preferred is (*S*)-5-(diphenylmethanesulfinylmethyl)-1,3-thiazole which can be represented by the following formula:

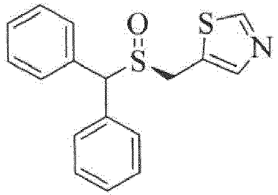

Accordingly, with the compounds according to formula (I), preferably containing the group of the general formula (IIa), more preferably (IIIa) or (IIIb), in particular preferred (S)-4-(diphenylmethanesulfinylmethyl)-2-cyclopropyl-1,3-thiazole and (S)-5-(diphenylmethane-sulfinylmethyl)-1,3-thiazole, a novel chemotype of compounds, in particular with motivation and/or reference memory enhancing effects, in particular with motivation and reference memory enhancing effects, is provided with the present invention that can be used for improving motivation and/or cognitive functions, such as learning capabilities, in particular reference memory, both with healthy, e.g. healthy aging, and impaired, e.g. impaired aging, individuals. Moreover, with the S enantiomers of the compounds of formula (I), in particular (S)-4-(diphenylmethanesulfinylmethyl)-2-cyclopropyl-1,3-thiazole and (S)-5-(diphenylmethanesulfinylmethyl)-1,3-thiazole, also patients with dementing disorders where motivation and/or reference memory enhancing is of benefit can be effectively treated. That is, with the S enantiomeric form of the compounds of the present invention as specified with formula (I), preferably containing the group of the general formula (IIa), more preferably (IIIa) or (IIIb), motivation and/or cognitive functions such as learning capabilities and in particular the motivation and reference memory in human individuals, in particular in aging individuals can be improved. Hence, the present invention provides a compound according to formula (I), in particular the S enantiomer of 4-(diphenylmethanesulfinylmethyl)-2-cyclopropyl-1,3-thiazole and 5-(diphenylmethane-sulfinylmethyl)-1,3-thiazole, for use in improving motivation and/or cognitive functions and in particular the motivation and reference memory, in particular motivation and reference memory, in human individuals, in particular in aging individuals. However, in general with the present invention compounds of formula (I) as defined herein are provided for use in therapy, i.e. for use as a medicament, and in particular for treating age-related cognitive decline, more in particular for treating age-related cognitive decline according to 2018 ICD-10-CM Diagnosis Code R41.81.

It is of particular interest that with the compounds of formula (I), preferably containing the group of the general formula (IIa), preferably (IIIa) or (IIIb), in particular preferred the S enantiomer of 4-(diphenylmethanesulfinylmethyl)-2-cyclopropyl-1,3-thiazole and 5-(diphenylmethanesulfinylmethyl)-1,3-thiazole, improvements in terms of motivation and/or cognitive functions such as learning capabilities, in particular reference memory, in particular improvements in terms of motivation and cognitive functions, can be accomplished with human individuals who do not have reference memory deficits and/or cognitive defects. Accordingly, these results can be obtained with individuals who do not have reference memory deficits and/or cognitive defects caused by diseases of the brain. The compounds according to the present invention can thus also be administered to and are also effective with healthy individuals to improve motivation and/or referene memory, in particular motivation and reference memory, and/or other cognitive abilities such as learning capabilities (or to overcome respective deficits). Accordingly, the present invention therefore also relates to the use of a compound according to the present invention for the improvement of motivation and/or cognitive deficits, in particular the reference memory, more in particular motivation and reference memory, in healthy individuals, in particular in healthy aging individuals.

In addition, with the compounds of formula (I), preferably containing the group of the general formula (IIa), preferably (IIIa) or (IIIb), in particular preferred 4-(diphenylmethanesulfinylmethyl)-2-cyclopropyl-1,3-thiazole and 5-(diphenylmethane-sulfinylmethyl)-1,3-thiazole, improvements in terms of motivation and/or cognitive functions such as learning capabilities, in particular motivation and cognitive functions, more in particular motivation and reference memory, and even more preferably motivation and reference memory, can also be accomplished with human individuals who have reference memory deficits and/or cognitive defects, in particular reference memory deficits, caused by diseases of the brain. In this regard, a patient can be treated with the compounds according to formula (I), containing the group of the general formula (IIa), preferably (IIIa) or (IIIb), in particular preferred 4-(diphenylmethanesulfinylmethyl)-2-cyclopropyl-1,3-thiazole and 5-(diphenylmethane-sulfmylmethyl)-1,3-thiazole, who has reference memory deficits and/or cognitive defects, in particular and/or reference memory deficits, caused by Alzheimer; Down syndrome; vascular cognitive impairment; stroke; frontotemporal dementia; behavioural, semantic or progressive aphasia type dementia; dementia with Lewy bodies; subcortical dementias; Parkinson's disease dementia; alcohol related dementia; dementia caused by traumatic brain injury; Huntington's disease related dementia; AIDS-related dementia; attention deficit disorders; or schizophrenia, or who has any form of cognitive impairment.

The (S)-enantiomers of the compounds according to formula (I) of the present invention, in particular (S)-4-(diphenylmethanesulfinylmethyl)-2-cyclopropyl-1,3-thiazole and (S)-5-(diphenylmethanesulfinylmethyl)-1,3-thiazole, can be used alone or in combination with other cognitive enhancing compounds such as rivastigmin, donepezil, galanthamin, clozapine, risperidone, memantine, olanzapine, aripiprazole, quitiapine, clozapine, D1 agonists, nicotinic alfa 7-agonists, d-serine, d-cycloserine. PDE2, 4,9 inhibitors, AMPA agonists, lamotrigine, n-desmethylclozapine, mGlu receptor agonists, GABA A receptor agonists, muscarinic 1 and 4 receptor agonists, to treat lack of motivation and/or cognitive impairments and improve motivation and/or reference memory, in particular motivation and reference memory.

The compounds according to the present invention are particularly useful for treating patients having reference memory deficits and/or cognitive defects due to particular demands in healthy and aging individuals, diseases of the brain, mental disorders or cognitive deficits, especially patients having Alzheimer, Down syndrome, stroke (vascular cognitive impairment), frontotemporal dementia, behavioural, semantic and progressive aphasia type dementia, dementia with Lewy bodies, subcortical dementias, and Parkinson's disease dementia, alcohol related dementia, dementia caused by traumatic brain injury, Huntington's disease related dementia, AIDS-related dementia, attention deficit disorders; or schizophrenia.

The compound according to formula (I), preferably containing the group of the general formula (IIa), preferably (IIIa) or (IIIb), in particular 4-(diphenylmethanesulfinylmethyl)-2-cyclopropyl-1,3-thiazole and 5-(diphenylmethanesulfinylmethyl)-1,3-thiazole or (IIa), can be administered to a patient having or being at risk to develop any of the disorders referred to above in an effective amount. It is also possible to administer the compound to healthy individuals for improving motivation and/or cognitive functions such as learning capabilities, in particular motivation and reference memory.

According to a preferred embodiment, it is convenient to administer the compound according to the present invention, in particular 4-(diphenylmethanesulfinylmethyl)-2-cyclopropyl-1,3-thiazole and 5-(diphenylmethanesulfinylmethyl)-1,3-thiazole, by a single dosage unit form, especially as a capsule or a tablet. A preferred formulation comprises a soft gelatine capsule containing at least one compound according to formula (I) dissolved in oil within the capsule with one or more emulsifying agents. Accordingly, gelatine capsules are preferred which comprise one or more emulsifying agents and also at least one compound according to formula (I) dissolved in at least one oil.

Preferred dosages comprise administration dosages of about 1 mg/kg to about 25 mg/kg body weight. Suitable dosage forms also comprise formulations comprising at least one compound according to formula (I) in an amount of about 0.1 mg to about 10 g, preferably of about 1 mg to about 1 g, and more preferably of about 10 mg to about 200 mg.

According to a preferred embodiment, the S enantiomers of the compound of formula (I) according to the present invention, in particular (S)-4-(diphenylmethanesulfinylmethyl)-2-cyclopropyl-1,3-thiazole and (S)-5-(diphenylmethanesulfinylmethyl)-1,3-thiazole, can be part of formulations which contain at least or can be administered with one or more compounds, also referred to as co-agents, selected from the group consisting of Rivastigmin, Donepezil, Galanthamin, clozapine, risperidone, memantine, olanzapine, aripiprazole, quitiapine, clozapine, D1 agonists, nicotinic alfa 7, agonists, d-serine, d-cycloserine, PDE2,4,9 inhibitors, AMPA agonists, Lamotrigine, n-desmethylclozapine, mGlu receptor agonists, GABA A receptor agonists, and Muscarinic 1 and 4 agonists or other potential cognitive enhancing compounds.

The object of the present invention has also been solved by the use of the (*S*)-enantiomer of a compound according to formula (I), in particular the S enantiomer of 4-(diphenylmethanesulfinylmethyl)-2-cyclopropyl-1,3-thiazole and 5-(diphenylmethane-sulnnylmethyl)-1,3-thiazole, in the manufacture of a medicament for the improvement of motivation and/or cognitive functions in human individuals, in particular for the improvement of motivation and cognitive functions in human individuals, more in particular the motivation and/or reference memory, in particular motivation and reference memory.

According to another aspect of the present invention the S enantiomer of a compound according to formula (I), in particular the S enantiomer of 4-(diphenylmethanesulfinylmethyl)-2-cyclopropyl-1,3-thiazole and 5-(diphenylmethane-sulfinylmethyl)-1,3-thiazole, has been found for use in inhibiting DAT-mediated dopamine reuptake in the brain of a mammal, in particular a human individual, and also in the manufacture of a medicament for inhibiting DAT-mediated dopamine reuptake in the brain of a mammal, in particular of a human individual.

Moreover, the object of the present invention has also been solved by a pharmaceutical preparation, also referred to herein by pharmaceutical composition, comprising at least one compound according to formula (I), in particular 4-(diphenylmethanesulfinylmethyl)-2-cyclopropyl-1,3-thiazole and 5-(diphenylmethanesulfinylmethyl)-1,3-thiazole, and at least one pharmaceutically acceptable carrier and/or diluent. Thus, according to another aspect, the present invention relates to a pharmaceutical preparation comprising a compound according to the present invention and a pharmaceutically acceptable carrier or diluent.

Pharmaceutical "carriers", "diluents" as well as "excipients" are substances to be admixed in a pharmaceutical composition as auxiliary substances to the compound according to the present invention or other compounds with pharmaceutical effect (e.g. that have also neurological or behavioural effects). These auxiliary substances do not have a pharmaceutical effect on their own (they are no "active substance" per se) but may assist to optimise the effectivity of the compound according to the present invention. The substances used as "carriers", "diluents" and excipients" can often be used interchangeably (i.e. that substances that are used as "diluents" may also serve as "excipients" and/or "carriers").

In general, "carriers" are substances that improve the delivery and the effectiveness of drugs; "diluents" are substances that dilute the active substance in a pharmaceutical preparation; "excipients" are substances that are admixed to the pharmaceutical formulation for defining its releasing properties.

All such "carriers", "diluents" and "excipients" (i.e. auxiliary substances, sometimes also the term "excipient" is used as the general term including all such substances) are, as already stated, inactive substances formulated alongside the active ingredient ("API") of a medication, for the purpose of bulking-up formulations that contain potent active ingredients (thus often also referred to as "bulking agents", "fillers", or "diluents"). Bulking up allows convenient and accurate dispensation of a drug substance when producing a dosage form. They also can serve various therapeutic-enhancing purposes, such as facilitating drug absorption or solubility, or other pharmacokinetic considerations. Such substances can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients, carriers and diluents also depends upon the route of administration and the dosage form, as well as the active ingredient and other factors. Such substances e.g. serve as antiadherents, binders, coatings, disintegrants, fillers, flavours, colours, lubricants, glidants, sorbents, preservatives, sweeteners, etc.

Drug "carriers" are substances that serve as mechanisms to improve the delivery and the effectiveness of drugs. Drug carriers are usually used in sundry drug delivery systems such as: controlled-release technology to prolong in vivo drug actions; decrease drug metabolism, and reduce drug toxicity.

Carriers are generally also used in designs to increase the effectiveness of drug delivery to the target sites of pharmacological actions.

Drug "diluents" are usually substances that simply dilute or reconstitute a pharmaceutical composition (e.g. after storage in dry form).

The pharmaceutical composition with the at least one S enantiomer of the compound according to formula (I), in particular (S)-4-(diphenylmethanesulfinylmethyl)-2-cyclopropyl-1,3-thiazole and (S)-5-(diphenylmethanesulfinylmethyl)-1,3-thiazole, usually comprises said compound in an amount of about 0.01 (%w/w) to about 80 (%w/w) in a solid composition or about 0.001 (%w/v) to about 80 (%w/v) in a liquid composition. Preferably the compound according to the present invention is present in an amount of about 0.1 (%w/w) to about 50 (%w/w) in a solid composition or about 0.01 (%w/v) to about 10 (%w/v) in a liquid composition, especially of about 1 (%w/w) to about 0 (%w/w) in a solid composition or about 0.1 (%w/v) to about 5 (%w/v) in a liquid composition.

As already stated, preferably, the S enantiomers of the compounds according to formula (I), in particular (S)-4-(diphenylmethanesulfinylmethyl)-2-cyclopropyl-1,3-thiazole and (S)-5-(diphenylmethanesulfinylmethyl)-1,3-thiazole, are provided as a pharmaceutical composition, especially as a pharmaceutical single unit dosage form. Pharmaceutical compositions and single unit dosage forms provided herein comprise a prophylactically or therapeutically effective amount of one or more compounds according to formula (I) and optionally also one or more additional prophylactic or therapeutic agents (e.g., in particular a compound provided herein, or other prophylactic or therapeutic agent), and one or more pharmaceutically acceptable carriers or excipients or diluents. In a specifically preferred embodiment and in this context, the term "pharmaceutically acceptable" means approved by a regulatory agency of the Federal or a state government or listed in the European or U.S. Pharmacopeia or other generally recognized pharmacopeia for use in animals, and more particularly in humans.

The term "carrier" refers in one embodiment to a diluent, adjuvant (e.g., Freund's adjuvant (complete and incomplete)), or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a particular carrier when the pharmaceutical composition is administered intravenously. Saline solutions and aqueous dextrose and glycerol solutions can also be employed as liquid carriers, particularly for injectable solutions.

In one embodiment, pharmaceutical compositions and dosage forms comprise one or more excipients. Suitable excipients are well-known to those skilled in the art of pharmacy, and non-limiting examples of suitable excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form usually depends on a variety of factors well known in the art including, but not limited to, the way in which the dosage form will be administered to a patient and the specific active ingredients in the dosage form. The composition or single unit dosage form, if desired, can also contain minor amounts of wetting or emulsifying agents, or pH buffering agents.

Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

Examples of suitable excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatine, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxy-propyl methyl cellulose, (e.g., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions provided herein is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Disintegrants can be used in the compositions of the present invention to provide solid dosage forms such as tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms provided herein. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Suitalbe disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Suitable lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

The active ingredients described herein such as the S enantiomers of the compounds according to formula (I) and/or the co-agents provided herein can be administered by controlled release means or by delivery devices that are well known to those of ordinary skill in the art. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients. Thus provided are single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

Parenteral dosage forms can be administered to patients by various routes including, but not limited to, oral, subcutaneous, intravenous (including bolus injection), intramuscular, and intra-arterial. Because their administration typically bypasses patients' natural defences against contaminants, parenteral dosage forms are in certain embodiments sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, com oil, cottonseed oil, peanut oil, sesame oil, ethyl oleate, isopropyl myristate, and benzyl benzoate.

According to a preferred embodiment, the pharmaceutical preparation of the present invention further comprises one or more compounds selected from the group consisting of Rivastigmin, Donepezil, Galanthamin, clozapine, risperidone, memantine, olanzapine, aripiprazole, quitiapine, clozapine, D1 agonists, nicotinic alfa 7, agonists, d-serine, d-cycloserine, PDE2,4,9 inhibitors, AMPA agonists, Lamotrigine, n-desmethylclozapine, mGlu receptor agonists, GABA A receptor agonists, and Muscarinic 1 and 4 agonists or other potential cognitive enhancing compounds, agents.

Preferred dosages of the compound according to formula (I), in particular 4-(diphenylmethanesulfinylmethyl)-2-cyclopropyl-1,3-thiazole and 5-(diphenylmethanesulfinylmethyl)-1,3-thiazole, are 0.1 mg to 10 g, preferably of 1 mg to 1 g, especially 10 mg to 200 mg, of the compound according to the present invention. As already stated, the invention relates to the use of a composition according to formula (I) for the manufacture of a medicament. And, the invention also relates to the use of a composition comprising at least one compound according to formula (I) for the manufacture of a remedy i.e. pharmacological treatment or prevention of cognitive impairment, dementia and/or reference memory deficits.

The S enantiomers of the compound of the formula (I), in particular (S)-4-(diphenylmethanesulfinylmethyl)-2-cyclopropyl-1,3-thiazole and (S)-(5-(diphenylmethanesulfinylmethyl)-1,3-thiazole, can be administered to humans, rodents other mammals, as therapeutics per se, and as mixtures with one another or in the form of pharmaceutical preparations which allow enteral or parenteral use and which as active constituent contain an effective dose of the compound of formula (I). The effective dose range can easily be adapted to the severity and the need of the individual patient. Preferred doses are from 1 mg to 10 mg/kg body weight, in addition to customary pharmaceutically innocuous excipients and additives.

The proposed therapeutics can be administered orally, e.g. in the form of pills, tablets, coated tablets, sugar coated tablets, hard and soft gelatin capsules, solutions, syrups, emulsions or suspensions. Administration, however, can also be carried out rectally, e.g. in the form of suppositories, or parenterally, e.g. in the form of injections or infusions or percutaneously, e.g. in the form of ointments, creams or tinctures.

In addition to the active compound of formula (I), the pharmaceutical composition can contain further customary, usually inert carrier materials or excipients, in particular as outlined above. Thus, the pharmaceutical preparations can also contain additives, such as, for fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants.

The compounds of the present invention can be used in the form of one substance alone or in combination with other active compounds - for example with remedies already known to improve reference memory of healthy individuals, in particular of healthy aging individuals.

The invention also provides a pharmaceutical composition comprising compounds of formula (I), in particular 4-(diphenylmethanesulfinylmethyl)-2-cyclopropyl-1,3-thiazole and 5-(diphenylmethanesulfinylmethyl)-1,3-thiazole, in free form or in the form of pharmaceutically acceptable formulations and together with pharmaceutically acceptable diluents or carriers.

The S enantiomer of the compounds of formula (I), in particular 4-(diphenylmethanesulfinylmethyl)-2-cyclopropyl-1,3-thiazole and 5-(diphenylmethanesulfinylmethyl)-1,3-thiazole, and also the pharmaceutically acceptable salt or solvate thereof can be used as the active principle for improvement of motivation and/or cognitive functions such as learning capabilities, in particular improvement of motivation and cognitive functions, and more in particular for the enhancement of motivation and reference memory, also in healthy or unimpaired individuals.

The compound according to the present invention is defined by formula (I), wherein the connection between the methylene group and the heterocyclic compound R_{TA} can be with any position where there is no R₃ and where there is a free valence available for the formation of a covalent bond. As well, the connection between R₃ with the heterocycle can be with any position where the connection of the methylene group is not present and where there is a free valence available for the formation of a covalent bond. R₃ is present in formula (IIa) independently 1, or 2 times, equal or independently. R₃ preferably is hydrogen or an alkyl group, in particular methyl, ethyl, iso-propyl, tert-butyl, or a hydroxyalkyl group, in particular hydroxymethyl, or a cycloalkyl group, or a heterocycloalkyl group, or a dialkylamino group, in particular dimethylamino, or a heterocycloalkyl group, in particular morpholino, piperazino or methylpiperazino.

Compounds having infinite chains consisting for instance of repeating R₁, R₂, R₃ units and the like are not encompassed by this invention.

Constituents which are optionally substituted as stated herein, may be substituted, unless otherwise noted, at any chemically possible position.

The invention also provides a pharmaceutical composition comprising a compound of Formula (I), in particular 4-(diphenylmethanesulfinylmethyl)-2-cyclopropyl-1,3-thiazole and 5-(diphenylmethanesulfinylmethyl)-1,3-thiazole, in free form or in the form of pharmaceutically acceptable salts and/or physiologically functional derivatives, together with a pharmaceutically acceptable diluent or carrier therefore. The present invention also relates to a compound of Formula (I) and physiologically acceptable salts or physiologically functional derivatives thereof. The compounds of the present invention according to Formula (I), in particular 4-(diphenylmethanesulfinylmethyl)-2-cyclopropyl-1,3-thiazole and 5-(diphenylmethanesulfinylmethyl)-1,3-thiazole), are many times more effective and also many times more selective than for example Modafinil (2-(diphenylmethyl) sulfinyl acetamide).

The present invention also contemplates a pharmaceutical composition comprising the S enantiomer of the compound of the general Formula (I), in particular (S)-4-(diphenylmethanesulfinylmethyl)-2-cyclopropyl-1,3-thiazole and (S)-5-(diphenylmethanesulfinylmethyl)-1,3-thiazole, in free form and also to the use of in free form and physiologically functional derivatives, together with pharmaceutically acceptable diluents or carriers.

The present invention also relates to the use of the S enantiomer of the compound of the Formula (I), in particular 4-(diphenylmethanesulfinylmethyl)-2-cyclopropyl-1,3-thiazole and 5-(diphenylmethanesulfinylmethyl)-1,3-thiazole, and also to the use of physiologically functional derivatives in the manufacture of a medicament for the motivation and/or cognitive enhancement, in particular for the motivation and cognitive enhancement, of healthy individuals or aging individuals or of individuals with cognitive impairments, or for the treatment of a disease or a therapeutic indication in which improvement of motivation and/or cognitive functions, in particular for the motivation and cognitive enhancement, and more in particular of the motivation and reference memory is beneficial.

Preferably, the pharmaceutical composition according to the present invention comprises the S enantiomer of the compound as defined by Formula (I), in particular (S)-4-(diphenylmethanesulfinylmethyl)-2-cyclopropyl-1,3-thiazole and (S)-5-(diphenylmethanesulfinylmethyl)-1,3-thiazole, in free form and a pharmaceutically acceptable diluent or carrier for use in the treatment of healthy individual or aging individuals or treatment of a disease or medical condition accompanied by reference memory deficits.

However, in general with the present invention also a pharmaceutical composition has been provided comprising the S enantiomer of the compound according to formula (I) and at least one pharmaceutically acceptable carrier and/or diluent, as defined herein, for use in therapy, i.e. for use as a medicament, in particular for treating age-related cognitive decline, more in particular for treating age-related cognitive decline according to 2018 ICD-10-CM Diagnosis Code R41.81.

The present invention also relates to a pharmaceutical composition comprising a compound as defined herein in free form or in the form of a pharmaceutically acceptable diluents or carrier for use in the treatment of healthy individuals or aging individuals or for the treatment of a disease or medical condition accompanied by reference memory deficits.

Accordingly, the problem of the present invention is also solved by the S enantiomer of a compound according to Formula (I), in particular (S)-4-(diphenylmethanesulfinylmethyl)-2-cyclopropyl-1,3-thiazole and (S)-5-(diphenylmethanesulfinylmethyl)-1,3-thiazole, for use in improving motivation and/or cognitive functions such as learning capabilities, in particular motivation and cognitive functions, in human individuals, in particular in aging individuals. Preferably, said compound for use is used in improving motivation and the reference memory in human individuals. The compound can in particular be used for improving motivation and/or cognitive functions such as learning capabilities, in particular motivation and cognitive functions, and more in particular motivation and reference memory, with human individual who do not have defects of cognitive functions and/or reference memory deficits, or any such defects or deficits caused by diseases of the brain. Alternatively, the S enantiomers of the compound according to Formula (I), in particular (S)-4-(diphenylmethanesulfinylmethyl)-2-cyclopropyl-1,3-thiazole and (S)-5-(diphenylmethanesulfinylmethyl)-1,3-thiazole, can be used for human individuals who have defects of cognitive functions and/or reference memory deficits caused by diseases of the brain. The causes for such defects of cognitive functions and/or reference memory deficits are those as mentioned above.

Accordingly, the problem of the present invention is also solved by the use of the S enantiomer of a compound according to Formula (I) for the preparation of a medicament for improving motivation and/or cognitive functions such as learning capabilities, in particular motivation and cognitive functions, in human individuals, in particular in aging individuals. Preferably, said prepared medicament is used in improving motivation and the reference memory in human individuals. Said prepared medicament can in particular be used for improving motivation and/or cognitive functions such as learning capabilities, in particular motivation and cognitive functions, and more in particular motivation and reference memory, with human individual who do not have defects of cognitive functions and/or reference memory deficits, or any such defects or deficits caused by diseases of the brain. Alternatively, said medicament prepared by use of the S enantiomer of the compound according to Formula (I), in particular 4-(diphenylmethanesulfinylmethyl)-2-cyclopropyl-1,3-thiazole and 5-(diphenylmethanesulfinylmethyl)-1,3-thiazole, can be used for treating human individuals who have defects of cognitive functions and/or reference memory deficits caused by diseases of the brain. The causes for such defects of cognitive functions and/or reference memory deficits are those as mentioned above.

It has also surprisingly been found that the S enantiomeric form of the compound according to Formula (I), in particular *(S)-5-*((benzhydrylsulfinyl)methyl)thiazole ((*S*)-5-(diphenylmethanesulfinylmethyl)-1,3-thiazole), can be used in improving the reference memory and/or motivation and in particular reference memory and motivation, more in particular with individuals suffering from depression or depressive disorders.

"Pharmaceutically acceptable salt" refers to any salt of a compound provided herein which retains its biological properties and which is not toxic or otherwise undesirable for pharmaceutical use. Such salts may be derived from a variety of organic and inorganic counter-ions well known in the art and include: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, /ert-butylacetic, lauryl sulfuric, gluconic, benzoic, glutamic, hydroxynaphthoic, salicylic, stearic, cyclohexylsulfamic, quinic, muconic acid and the like acids; or (2) salts formed when an acidic proton present in the parent compound either (a) is replaced by a metal ion, e.g., an alkali metal ion, an alkaline earth ion or an aluminum ion, or alkali metal or alkaline earth metal hydroxides, such as sodium, potassium, calcium, magnesium, aluminum, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like. Specifically preferred salt forms are hexaflorophosphate and chloride salts.

Such salts further include, by way of example only, sodium, potassium, calcium, magnesium, ammonium, tetraalkylammonium and the like, and when the compound contains a basic functionality, salts of non-toxic organic or inorganic acids, such as hydrochloride, hydrobromide, tartrate, mesylate, besylate, acetate, maleate, oxalate and the like. The term "physiologically acceptable cation" refers to a non-toxic, physiologically acceptable cationic counterion of an acidic functional group. Such cations are exemplified by sodium, potassium, calcium, magnesium, ammonium and tetraalkylammonium cations and the like.

The compounds according to the present invention may also be provided as solvates. "Solvate" refers to a compound provided herein or a salt thereof that further includes a stoichiometric or non-stoichiometric amount of solvent bound by non-covalent intermolecular forces. Where the solvent is water, the solvate is a hydrate.

The invention is further described by the following examples given in the figures, yet without being restricted thereto.
- Fig. 1: shows behavioral performance (reference memory index, RMI) of young (A) and old (B) rats; and
- Fig. 2: shows the results of the FR5/chow feeding choice experiment;

### EXAMPLES

### A. Syntheses

### 1. Synthesis of 5-(chloromethyl)thiazole hydrochloride

5-(Hydroxymethyl)thiazole (0.63 g, 5.5 mmol) was dissolved in 20 mL of dichloromethane in a 100 mL round-bottomed flask, which was subsequently placed in an ice bath set up on a magnetic stirrer. Thionyl chloride (0.40 mL, 5.5 mmol) was carefully dripped in via a syringe and the reaction was allowed to proceed overnight. The reaction mixture was condensed on a rotary evaporator and the dark yellow grainy substance thus obtained was additionaly dried in high vacuum to remove lingering thionyl chloride as well as water and afforded 0.93 g (5.5 mmol) of a highly hygroscopic yellow powdery product in quantitative yield.

### 2. Synthesis of [(diphenylmethyl)sulfanyl]methanimideamide hydrobromide

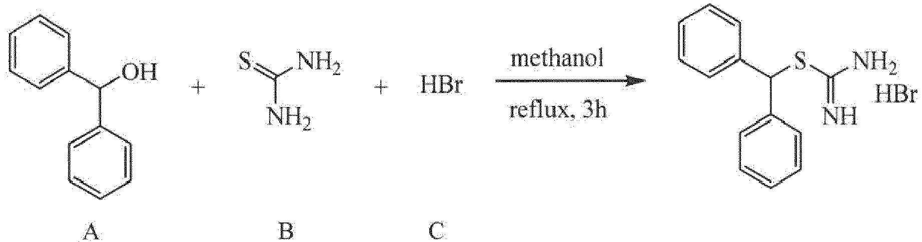

Diphenylmethanol (1.29 g, 7 mmol) was dissolved in 30 mL of methanol in a 100 mL three-necked round-bottomed flask, thiourea (0.61 g, 8 mmol) was added and the reaction mixture was refluxed (external temperature 87 to 90°C) for half an hour until homogeneity was achieved. Hydrogen bromide (3.64 mL, 32.2 mmol) was added in a dropwise fashion over the course of half an hour, and the reaction mixture was further refluxed for 3 hours.

The reaction mixture was allowed to cool down to room temperature, it was poured from the three-necked flask into a 100 mL round-bottomed flask and methanol was evaporated. The remaining substance was suspended in 30 mL of dichloromethane, stirred for half an hour (batch extraction of remaining diphenylmethanol) and filtered through a Buechner funnel. The solid residue was washed with additional 30 mL of dichloromethane and suspended afterwards in 30 mL of water, stirred for half an hour (batch extraction of unreacted thiourea) and filtered through a Buechner funnel. The residue was washed with 30 mL of water and dried. 1.62 g (5 mmol) of white, fine powdered product was obtained, amounting to isolated yield of 71%.

### 3. Synthesis of 5-((diphenylmethylthio)-methyl)thiazole

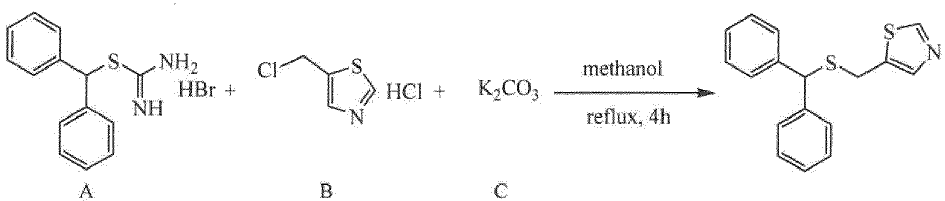

Compound A (1.62 g, 5 mmol) was dissolved in 30 mL of methanol in a 100 mL round-bottomed flask mounted of a magnetic stirrer. 5-(Chloromethyl)thiazole hydrochloride (0.93 g, 5.5 mmol) was added and dissolved, potassium carbonate (3.45 g, 25 mmol) was subsequently introduced and the reaction mixture was refluxed for 4 hours (external temperature was 87 to 90°C). The majority of potassium carbonate was disposed off by filtering of the hot reaction mixture on through a Buechner funnel; methanol was evaporated from the filtrate. The remaining substance was suspended in 50 mL of water, and organic products were extracted with ethyl acetate (75 mL, 3 portions). The extracts were pooled, dried with anhydrous Na₂SO₄, filtered and condensed under reduced pressure to afford an oily mixture which was purified by column chromatography on silica gel (2.5% solution of methanol in dichloromethane was used as eluent). 1.25 g (4.25 mmol) of the desired product was obtained in this manner, which corresponds to isolated yield of 84%.

### 4. Synthesis of (S)-5((diphenylmethanesulfinyl)-methyl-1,3-thiazole) (Compound C1)

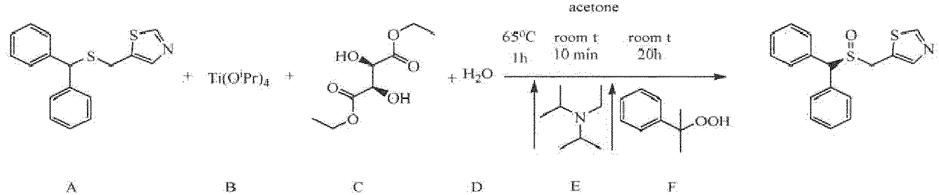

5-((Benzhydrylthio)methyl)thiazol (1.25g, 4.25 mmol) was dissolved in 25 mL of acetone in a 100 mL round-bottomed flask. Titanium isopropoxide (0.37 mL, 1.25 mmol), (S,S)-(-) diethyl tartrate (0.43 mL, 2.5 mmol) and water (11.25 µL, 0.625 mmol) were added to the mixture, which was stirred for 5 minutes to achieve homogeneity, upon which the flask was equipped with reflux condenser and the mixture was stirred for an hour at 65°C (external temperature). The mixture was given 15 minutes to cool down to room temperature, N,N-Diisopropylethylamine (0.15 mL, 0.85 mmol) was added and, after 10 minutes of stirring, Cumene hydroperoxide (0.78 mL, 4.25 mmol) was added and the reaction was allowed to proceed for 20 hours. Acetone was evaporated from the mixture and the oily orange-brown residue was purified by column chromatography on silica gel (2.5% solution of methanol in dichloromethane was used as eluent). Thus 0.99 g of ((*S*)-5-((diphenylmethanesulfinyl)-methyl-1,3-thiazole) (Compound C1) was obtained, which corresponds to a 74% yield.

### B. Experiments demonstrating motivation and cognitive enhancing effects

The compound of the present invention can be used for motivation and cognitive enhancement of reference memory of normal and aged individuals with and without a variety of human diseases.

The compounds of the present invention were demonstrated to have motivation and cognitive enhancing activity and more specifically motivation and reference memory enhancing activity in an animal model.

It has been suggested that animal models employing tests of effort-based decision making can be used to study functions that are related to aspects of human motivational dysfunction (Salamone et al. 2006, 2016a, b, c). This strategy has been validated by clinical research showing that patients with major depression, Parkinson's disease and other disorders show a low-effort bias when tested on effort-related choice procedures (Treadway et al. 2012; Yang et al. 2014; Chong et al. 2016; Barch et al. 2017).

### B-1. Holeboard Test:

### Animals and keeping conditions:

Male Sprague-Dawley rats (17 to 18 weeks and 22 to 23 months old), bred and maintained in the Core Unit of Biomedical Research, Division of Laboratory Animal Science and Genetics, Medical University of Vienna were used. The animals were transferred to a separate experimental room one week before the start of the experimental procedure and kept there throughout the experiment individually in standard Makrolon cages filled with autoclaved woodchips temperature: 22 ± 2°C; humidity: 55 ± 5%; 12 h artificial light/12 h dark cycle: light on at 7:00 am). The study was carried out according to the guidelines of the Ethics committee, Medical University of Vienna, and were approved by the Federal Ministry of Education, Science and Culture, Austria.

### Holeboard apparatus and training:

The holeboard (1 m × 1 m) was made of black plastic and was surrounded by translucent plexiglass walls. The walls were equipped with spatial cues. Distal cues were provided by room structures outside the board. Four out of sixteen regularly arranged holes (diameter and depth 7 cm) were baited (dustless precision pellets, 45 mg, Bioserv®). The pattern of baited holes remained the same during the entire test. To avoid olfactory orientation pellets were also present in an area below the board. Prior to the experiment for three days the rats were handled for 15 min to get familiarized to the experimenter, followed by two days of habituation to the hole-board during which the animals could explore the maze for 15 min each day with access to food pellets. The weight of each rat was reduced by food restriction to reach 85% of its initial body weight before the start of the experiment. Tap water was given ad libitum. Rats were trained for three days (five trials on day one, four trials on day 2 and a retention trial at day 3). Every trial lasted for 120 s or until all four pellets were found. The apparatus was cleaned with 0.1% Incidin® (Ecolab, Düsseldorf, Germany) between trials to remove odor cues. The interval between two successive trials for an individual was 20 min. A camera mounted on the room ceiling recorded the performance of the rats in the maze. The hole visits and removals of pellets were noted for each trial. To compare rats with similar levels of motivation, rats with less than 40-hole visits in total over the ten trials were excluded from the analysis. Reference memory errors were noted as the number of visits to the unbaited holes. The Reference Memory Index (RMI) was calculated using the formula (first + revisits of baited holes)/total visits of all holes. All behavioral training/testing was performed during the light phase of the light-dark cycle.

### Results:

### Behaviour in young males

A significant trial effect was found (F_{9,324}=22.00, p<0.001), but no trial x treatment interaction (F_{27,324}=0.93, p=0.569) and no overall treatment effect between groups in young males (F_{3,33}=0.75, P=0.528) in the memory indices.

### Behaviour in old-males

And, a significant trial effect was determined (F_{9,315}=15.18, p<0.001), but no trial x treatment interaction (F_{27,315}=1.38, p=0.103), however, an overall treatment effect between groups in the memory indices (F_{3,35}=3.29, p=0.032). Tukey-post hoc tests revealed significantly enhanced RMI in rats treated with compound C₁ at a dosage of 10 mg/kg body weight (p=0.021), whereas rats treated with 1mg (p=0.357) or 5 mg (p=0.171) performed similar as compared to controls.

Training session-specific analysis between vehicle (DMSO/Tween 80/saline solution) and 10 mg compound C₁ treated rats revealed a significantly enhanced RMI in treated rats at day 1 (F_{1,18}=5.40, p=0.032), a significant trial effect (F_{4,72}=9.41, p<0.001) and a trial x treatment interaction (F_{4,72}=3.95, p=0.006). At day 2 there was no significant trial effect (F_{3,54}=2.41, p=0.077), no significant trial x treatment interaction (F_{3,54}=1.42, p=0.248), but a significant treatment effect (F_{1,18}=18.02, p<0.001). At the retention trial at day 3 a higher RMI was found in treated rats (T= -3.40, df = 18, p=0.003) compared to controls.

### Differences in behaviour between young and old males

Young animals treated with 10 mg/kg bodyweight showed lower RMI than old males treated with the same dosage (F_{1,18}=5.01, p=0.038) over the entire training. A significant trial effect (F_{9,162}=16.86, p<0.001) was found, and no trial x age interaction (F_{9,162}=0.80, p=0.616).

Day specific analyses revealed a significant trial effect (F_{4,72}=15.24, p<0.001), but no trial x age interaction (F_{4,72}=1.04, p=0.393), and no age effect (F_{1,18}=2.04, p=0.170) at day 1. A significant trial effect (F_{3,54}=4.89, p=0.004), no trial x age interaction (F_{3,54}=0.86, p=0.466), but a significant age effect (F_{1,18}=6.71, p=0.018) could be determined at day 2. No significant differences in RMI between groups could be observed at the retention trial at day 3 (T=1.87, df=18, p=0.077).

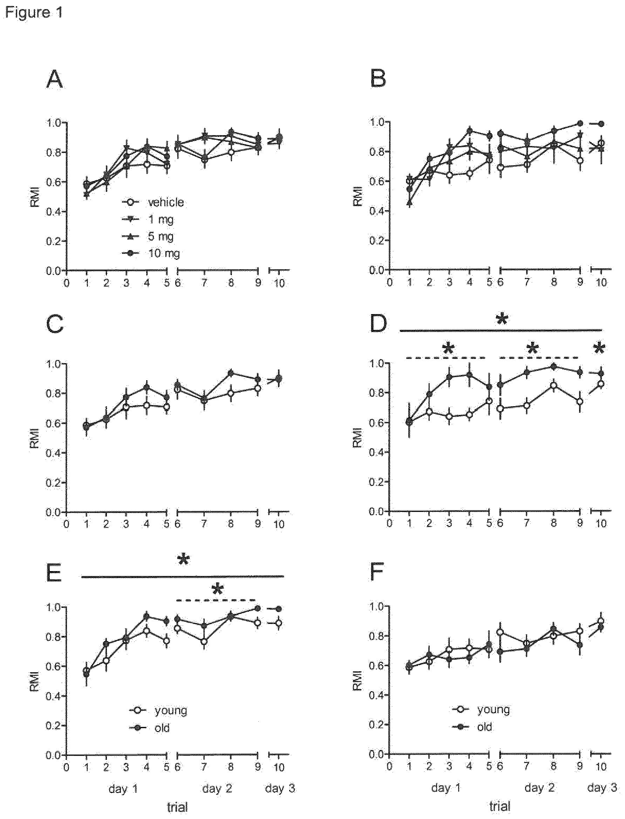

Fig. 1 shows behavioral performance (reference memory index, RMI) of young (A) and old (B) rats treated with 5 and 10 mg/kg body weight compound C1 and vehicle controls. The learning curves for vehicle and 10 mg/kg Compound C1 are given separately for young (C) and old (D) rats. RMI between young and old rats were compared for the 10 mg compound C1 dosage (E) and vehicle (F). Horizontal solid lines indicate statistical comparison over the entire training, dashed lines indicate day specific camparisons between groups. Asterisks indicate statistically significant differences. Given are the mean and SEM.

### B-2. Motivational studies showing the effect of compound C1:

Animals were offered a choice between high effort instrumental actions leading to highly valued reinforcers vs. low effort/low reward options. That is, effort-related choice behavior was used to model effort-related motivational dysfunctions in humans.

Rats were assessed using the fixed ratio 5/chow feeding choice test. Tetrabenazine (TBZ: 9,10-dimethoxy-3-(2-methylpropyl)-1,3,4,6,7,11b-hexahydrobenzo[a]quinolizin-2-one) was used as the vesicular monoamine transport blocker which inhibits DA storage, depletes brain DA, and reduces post-synaptic DA receptor signalling (Nunes et al. 2013). TBZ is known to shift choice behaviour and induce a low effort bias. And, it is also known that the reallocation of behaviour from high to low effort options that is produced by TBZ is not due to alterations in food preference or hedonic taste reactivity, reduced appetite or impairments in reference memory (Randall et al. 2012, 2014; Nunes et al. 2013; Correa et al. 2015; Pardo et al. 2015; Yohn et al. 2015a).

Tetrabenazine (1.0 mg/kg) shifted choice behavior, decreased lever pressing and increased chow intake. Compound C1 was co-administered at doses ranging from 3.0 to 24.0 mg/kg. The highest dose partially but significantly reversed the effects of tetrabenazine. Compound C1 was able to reverse the effort-related effects of TBZ. This suggests that compound C1 could be useful as a treatment for effort-related motivational dysfunction in humans.

The behavioural studies evaluated the motivational effects of compound C1 in rats using a test of effort-based choice behaviour, the concurrent fixed ratio 5 (FR5)/chow feeding choice task. Effort-related choice was studied using procedures that offer high effort options leading to highly valued reinforcers vs. low effort/low reward options (Salamone and Correa 2012; Salamone et al. 2016a,b,c). That is, the ability of compound C1 to attenuate the effort-related effects of TBZ in rats tested on the concurrent FR5/chow feeding choice task was evaluated.

### Animals and keeping conditions:

Adult male Sprague-Dawley rats (Envigo Sprague Dawley, Indianapolis, IN, USA; weights 275-299 g upon arrival) were pair-housed in a colony maintained at 23°C, with a 12-h light/dark cycle (lights on 07:00). Rats were food deprived to 85% of their free-feeding body weight for operant training and allowed modest growth throughout the experiment. Water was available ad libitum in the home cages. Animal protocols were approved by the University of Connecticut Institutional Animal Care and Use Committee, and the studies were conducted according to National Institutes of Health (NIH) guidelines.

### Behavioral Procedure: FR5/Chow Feeding Choice Task:

Behavioural sessions were conducted in operant chambers (28 X 23 X 23 cm3; Med Associates, Fairfax, VT) with 30 minute sessions 5 days/week. Rats were initially trained to lever press on a continuous reinforcement FR1 schedule (high-carbohydrate 45 mg pellets, BioServ, Frenchtown, NJ) and then shifted to the FR5 schedule. After 5 weeks of training on the FR5 schedule, chow was introduced. Weighed amounts of laboratory chow (Laboratory Diet, 5Poo Prolab RMH 3000, Purina Mills, St. Louis, MO; typically 15-20 g) were concurrently available on the floor of the chamber during the FR5/chow feeding choice task sessions. At the end of each 30 minute session, rats were immediately removed from the chambers, number of lever presses was recorded, and the amount of chow consumed was determined by weighing the remaining food (including spillage). Rats were trained on the FR5/chow feeding choice procedure for 5 weeks, after which drug testing began. On baseline and drug treatment days, rats consumed all of the operant pellets that were delivered during each session.

### Drug Treatments and Dose Selection:

Compound C1 ((*S*)-5((diphenylmethanesulfinyl)-methyl-1,3-thiazole)) was dissolved in dimethyl sulfoxide (DMSO), Tween 80, and 0.9% saline. The DMSO/Tween 80/saline solution was administered as the vehicle control. TBZ was obtained from Tocris Bioscience (El-lisville, MO) and was dissolved in DMSO, 0.9% saline, and titrated with HCl. The DMSO/saline solution was administered as the vehicle control. The TBZ dose was 1.0 mg/kg.

### Behavioural Experiments:

Trained rats (n=8) were administered either TBZ (1.0 mg/kg) or vehicle, and compound C1 (6.0, 12.0, and 24.0 mg/kg) or vehicle, on drug testing days. Rats received TBZ or vehicle 120 minutes before testing and compound C1 or vehicle 30 minutes before testing. The experiment used a within-groups design, with each rat receiving each drug treatment in a randomly varied order (one treatment per week, with none of the treatment sequences repeated across different animals). The following five treatment combinations were given:
TBZ vehicle + compound C1 vehicle;
1.0 mg/kg TBZ + compound C1 vehicle;
2.0 1/0 mg/kg TBZ + 6.0 mg/kg compound C1;
3.0 1.0 mg/kg TBZ + 12.0 mg/kg compound C1;
4.01.0 mg/kg TBZ+24.0 mg/kg compound C1.

Total number of lever presses and gram quantity of chow intake from the 30 minute sessions were analysed using repeated measures ANOVA. A statistical program (SPSS; version 2015) was used to perform all analyses. When there was a significant ANOVA, non-orthogonal planned comparisons were performed, using the overall error term to assess differences between each treatment and the control condition. The number of comparisons was restricted to the number of treatments minus one (Keppel, 1991).

### Results of FR5/Chow Feeding Choice Task:

Repeated measures ANOVA revealed that there was an overall significant effect of treatment with compound Ci on lever pressing [F(4,28)=34.625, p<0.001]. Planned comparisons showed that TBZ significantly decreased lever pressing compared to vehicle treatment (TBZ/vehicle vs. vehicle/vehicle [F(1,28) = 105.87, p < 0.001]). There also was a significant overall effects of treatment with compound C1 on chow intake [F(4,28)=27.280, p<0.001], and TBZ alone significantly increased chow intake relative to vehicle treatment (TBZ/vehicle vs. vehicle/vehicle [F(1,28) = 66.625, p < 0.001]). Additional planned comparisons revealed that co-administration of the dose of 24.0 mg/kg compound C1 with TBZ significantly attenuated the effects of TBZ on lever pressing (TBZ plus 24.0 mg/kg vs. TBZ/vehicle [F(1,28) = 13.2866, p < 0.01]) and chow intake (TBZ plus 24.0 mg/kg vs. TBZ/Veh [F(1,28) = 61.014, p < 0.001]).

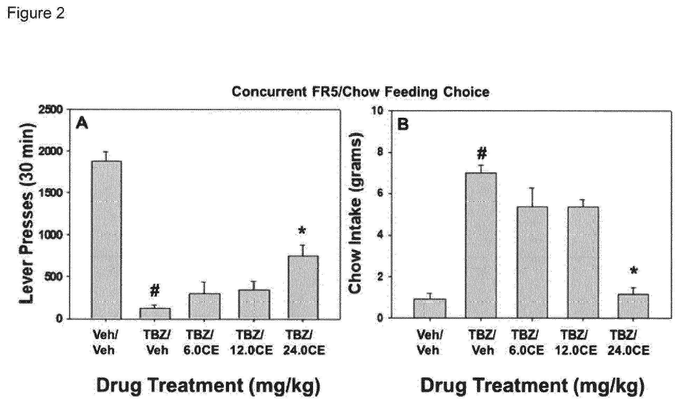

Figure 2 shows the results of the FR5/chow feeding choice experiment, namely TBZ shifted effort-based choice, decreasing lever pressing and increasing chow intake. That is, Fig. 2 is about the effects of the DAT blocker compound C1 on TBZ-induced changes in performance on the concurrent lever pressing/chow-feeding choice procedure. Rats (n=8) were administered vehicle plus vehicle (V/V), 1.0 mg/kg TBZ plus vehicle (TBZ/V), or TBZ plus 6.0, 12.0, or 24.0 mg/kg doses of compound C1.

In detail, Fig. 2A shows the mean (±SEM) number of lever presses (FR5/chow schedule) during the 30 minute session. For #p < 0.001, TBZ plus vehicle significantly differed from vehicle plus vehicle; and for *p < 0.01, TBZ plus 24.0 mg/kg Compound C1 significantly differed from TBZ plus vehicle. Fig. 2B shows the mean (±SEM) gram quantity of chow intake. For #p < 0.001, TBZ plus vehicle significantly differed from vehicle plus vehicle; and for **p < 0.001, TBZ plus 24.0 mg/kg Compound C1 significantly differed from TBZ plus vehicle.

### Reuptake inhibition assays:

[3H]5-HT (Hydroxytryptamine; 5-[1,2-3H[N]]; 27,8 Ci/ mmol), [3H]DA (Dihydroxy-phenylethylamine; 3,4-[ring-2,5,6-3[H]]-Dopamine; 36,6 Ci/mmol) and [3H]MPP+ (Methyl-4-phenylpyr-idinium iodide; 1-[methyl-3H]; 80 Ci/mmol) were purchased from Perkin Elmer, Boston, MA.

Effects of Compound C1 ((*S*)-5-(diphenylmethanesulfinylmethyl)-1,3-thiazole), (*R*)-5-(diphenylmethanesulfinylmethyl)-1,3-thiazole (in the following also referred to as Compound C2) and of racemic 5-(diphenylmethanesulfinylmethyl)-1,3-thiazole (in the following also referred to as Compound C3) on reuptake of their respective substrates were analyzed as described by Y.D. Aher et al. in Front. Behav. Neurosci. 10 (2016) 20 ("A novel heterocyclic compound CE-104 enhances spatial working memory in the radial arm maze in rats and modulates the dopaminergic system") and S. Sucic et al., in J. Biol. Chem. 285 (2010) 10924-10938 ("The N terminus of monoamine transporters is a lever required for the action of amphetamines"). HEK293 cells stably expressing human isoforms of the dopamine transporter (DAT), the nor-epinephrine transporter (NET) and the serotonin transporter (SERT) were used for reuptake inhibition assays. All cell lines were seeded on 96-well plates pre-coated with poly-D-lysine (PDL) (5 × 104 cells/well) 24 h prior to the experiment. Each well was washed with 100 µL of Krebs-HEPES buffer (KHB; 10 mM HEPES, 120 mM NaCl, 3 mM KCl, 2 mM CaCl2·2H2O, 2 mM MgCl2·6H2O, 5 mM D-(+)-Glucose mono-hydrate, pH 7.3). Cells were pre-incubated 5 min in KHB containing different dilutions (0.001 µM, 0.01 µM, 0.1 µM, 1 µM, 10 µM, 0.1 mM and 1 mM) of Compound C1, Compound C2 and Compound C3. Compound C1, Compound C2 and Compound C3 were separately dissolved first in 99.9% dimethyl sulfoxide (DMSO) and subsequently diluted in KHB. Afterwards, cells were incubated in KHB containing the same dilutions of Compound C1/Compound C2/Compound C3 with addition of 0.2 µM [3H]-dopamine (for HEK-DAT), 0.05 µM [3H]MPP+ (for HEK-NET) and 0.4 µM [3H]5-HT (for HEK-SERT). Incubation times were 1 min for HEK-DAT and HEK-SERT and 3 min for HEK-NET. For determination of non-specific uptake in HEK-DAT and HEK-NET 10 µM mazindole was used and 10 µM paroxetine was used for HEK-SERT. After incubation at room temperature, reactions were stopped by the addition of 100 µL of ice-cold KHB. Finally, cells were lysed with 300 µL of 1% SDS and released radioactivity was measured by a liquid scintillation counter (Tri-carb-2300TR, Perkin Elmer).

### Results:

Compound C1, Compound C2 and Compound C3 were characterized in HEK293 cells expressing cloned human transporters. A reuptake inhibition assay was used to determine the efficacy of Compound C1/Compound C2/Compound C3 to block the uptake of substrates by different monoamine transporters DAT, NET and SERT ([3H]DA, [3H]MPP+ and [3H]5-HT, respectively). It was found that Compound C1 very strongly inhibits DAT-mediated dopamine reuptake (IC50 = 2.8 µM ± 0.4) whilst inhibition of NET and SERT is negligible. It was found that Compound C3, i.e. the racemate, is much less effective in inhibiting DAT-mediated dopamine reuptake (IC50 = 4.6 µM ± 0.6), and that Compound C2, i.e. the R-enantiomer, showed a much lower effect in inhibiting DAT-mediated dopamine reuptake (IC50 = 22.3 µM ± 1.6).

Although modifications and changes maybe suggested by those skilled in the art, it is the intention of the applicant to embody within the patent warranted hereon all changes and modifications as reasonably and probably come within the scope of this contribution to the art. The features of the present invention which are believed to be novel are set forth in detail in the appended claims. The features disclosed in the description, the figures as well as the claims could be essential alone or in every combination for the realization of the invention in its different embodiments.

## Claims

1. (*S*)-enantiomer of the chemical compound having the general formula (I): 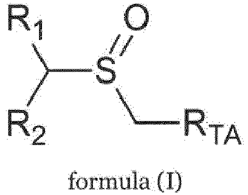 wherein R₁ and R₂ are, equal or independently, aryl, heteroaryl, fused aryl, fused heteroaryl, mono or multi-substituted aryl, mono or multi-substituted heteroaryl, mono or multi-substituted fused aryl, mono or multi-substituted fused heteroaryl; R_{TA} is a 2-1,3-, or 4-1,3- or 5-1,3-thiazole ring with the general Formula (IIa), 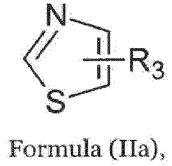 R₃ is present on the ring according to Formula (IIa) 1 or 2 times, equal or independently,
wherein R₃ is selected from the group consisting of hydrogen, alkyl, cycloalkyl, heterocycloalkyl, alkylamino, in particular dialkylamino, arylamino, in particular diarylamino, hydroxyalkylamino, alkoxy, arylalkyl, hydroxyalkyl, thioalkyl, haloalkyl, haloaryl, haloarylalkyl, haloalkoxy, mono or multi-substituted alkyl, mono or multi-substituted cycloalkyl, mono or multi-substituted heterocycloalkyl, mono or multi-substituted alkylamino, in particular dialkylamino, mono or multi-substituted arylamino, in particular diarylamino, mono or multi-substituted hydroxyalkylamino, mono or multi-substituted alkoxy, mono or multi-substituted arylalkyl, mono or multi-substituted hydroxyalkyl, mono or multi-substituted thioalkyl, mono or multi-substituted haloalkyl, mono or multi-substituted haloaryl, mono or multi-substituted haloarylalkyl, mono or multi-substituted haloalkoxy and carboxylate ester,
wherein substituted means substituted with a residue selected from the group consisting of alkyl, cykloalkyl, heterocycloalkyl, hydroxyalkyl, alkylthio, ether, hydroxyl, fluoride, chloride, bromide and iodide.

2. Compound according to claim 1, wherein
at least one of, in particular both of, R₁ and R₂ is/are aryl or substituted aryl, preferably phenyl or substituted phenyl, and/or wherein
R₃ is hydrogen or alkyl, in particular methyl, or oxyalkyl, in particular oxymethyl, or alkyl substituted with at least one residue selected from the group consisting of heterocycloalkyl, carboxylic acid, amide and ester, in particular methyl substituted with heterocycloalkyl, preferably heterocycloalkyl containing at least two heteroatoms

3. Compound according to claim 1 or claim 2, wherein
R_{TA} is a thiazole group, preferably a 2-thiazole group, in particular 2-thiazole, or a 4-thiazole group, in particular 2-methyl-4-thiazole.

4. Compound according to any one of the claims 1 to 3, wherein
R₁ and R₂ are, equal or independently, aryl or substituted aryl,
wherein R₃ is present on R_{TA} according to Formula (IIa) 2 times and selected from the group consisting of R₄, R₅, R₆ and R₇,
wherein R_{TA} is a 4-1,3-thiazole ring with the general Formula (IIIa) 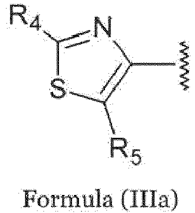 wherein R₄ is selected from the group consisting of oxirane, cyclopropyl, cyclobutyl, cyclopentyl and cyclohexyl and
wherein R₅ is selected from the group consisting of hydrogen, methyl, n-propyl, iso-propyl, fluoro, chloro, bromo and iodo,
or
wherein R_{TA} is a 5-1,3-thiazole ring with the general Formula (IIIb) 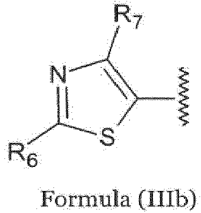 wherein R₆ is selected from the group consisting of hydrogen, methyl, n-propyl, iso-propyl, fluoro, chloro, bromo, iodo, cyclopropyl, cyclobutyl, cyclopentyl and cyclohexyl,
wherein R₇ is selected from the group consisting of hydrogen, methyl, n-propyl, iso-propyl, fluoro, chloro, bromo, iodo, cyclopropyl, cyclobutyl, cyclopentyl and cyclohexyl, and
wherein substituted means substituted with a residue selected from the group consisting of alkyl, cycloalkyl, heterocycloalkyl, hydroxyalkyl, alkylthio, ether, hydroxyl, fluoride, chloride, bromide and iodide.

5. Compound according to any one of the preceding claims, wherein
it is selected from the group consisting of
2-(diphenylmethanesulfinylmethyl)-1,3-thiazole,
4-(diphenylmethanesulfinylmethyl)-1,3-thiazole,
2-(diphenylmethanesulfinylmethyl)-4-methyl-1,3-thiazole,
5-(diphenylmethanesulfinylmethyl)-1,3-thiazole and
4-(diphenylmethanesulfinylmethyl)-2-methyl-1,3-thiazole,
5-(diphenylmethanesulfinylmethyl)-1,3-thiazole,
5-(diphenylmethanesulfinylmethyl)-2-chloro-1,3-thiazole,
5-(diphenylmethanesulfinylmethyl)-4-methyl-1,3-thiazole,
5-(diphenylmethanesulfinylmethyl)-4-chloro-1,3-thiazole,
5-(diphenylmethanesulfinylmethyl)-2-methyl-4-methyl-1,3-thiazole,
5-(diphenylmethanesulfinylmethyl)-2-methyl-4-chloro-1,3-thiazole,
5-(diphenylmethanesulfinylmethyl)-2-chloro-4-methyl-1,3-thiazole,
5-(diphenylmethanesulfinylmethyl)-2-cyclopropyl-1,3-thiazole,
4-(diphenylmethanesulfinylmethyl)-2-oxirane-1,3-thiazole,
4-(diphenylmethanesulfinylmethyl)-2-cyclopropyl-5-methyl-1,3-thiazole,
4-(diphenylmethanesulfinylmethyl)-2-cyclopropyl-5-chloro-1,3-thiazole,
4-(diphenylmethanesulfinylmethyl)-2-cyclopropyl-1,3-thiazole.

6. Compound according to any one of claims 1 to 5, wherein
it is 5-(diphenylmethanesulfinylmethyl)-2-methyl-1,3-thiazole or
5-(diphenylmethanesulfinylmethyl)-1,3-thiazole,

7. Compound according to any one of the preceding claims for use in therapy, in particular for treating age-related cognitive decline, more in particular for treating age-related cognitive decline according to 2018 ICD-10-CM Diagnosis Code R41.81.

8. Compound according to any one of claims 1 to 6 for use in improving motivation and/or cognitive functions, in particular learning capabilities, in human individuals, in particular motivation or motivation and cognitive functions.

9. Compound according to any one of claims 1 to 6 for use in improving the reference memory, in particular motivation and reference memory, in human individuals.

10. Compound for use according to claim 8 or 9, wherein
the human individual does not have defects of cognitive functions and/or reference memory deficits and/or motivation deficits, in particular does not have any reference memory deficits, caused by diseases of the brain.

11. Compound for use according to claim 10, wherein
the human individual does not have defects of cognitive functions and/or reference memory deficits and/or motivation deficits, in particular does not have any reference memory deficits.

12. Compound for use according to claim 8 or 9, wherein
the human individual has defects of cognitive functions and/or reference memory deficits and/or motivation deficits, in particular reference memory deficits, caused by diseases of the brain.

13. Compound for use according to claim 12, wherein
the human individual has defects of cognitive functions and/or reference memory deficits and/or motivation deficits, in particular reference memory deficits or motivation deficits or reference memory deficits and motivation deficits, caused by Alzheimer; Down syndrome; vascular cognitive impairment; stroke; frontotemporal dementia; behavioural, semantic or progressive aphasia type dementia; dementia with Lewy bodies; subcortical dementias; Parkinson's disease dementia; alcohol related dementia; dementia caused by traumatic brain injury; Huntington's disease related dementia; AIDS-related dementia; attention deficit disorders; reference memory deficiencies related to ageing; reference memory disorders related to viral infections of the brain; or schizophrenia.

14. Compound for use according to claim 13, wherein
the motivation deficits are caused by depression or depressive disorders.

15. Compound for use according to any one of claims 7 to 14, wherein
the human individual is an aging individual.

16. Compound according to any one of claims 1 to 6 for use in for use in inhibiting DAT-mediated dopamine reuptake in the brain of a human individual.

17. Use of a compound according to any one of claims 1 to 6 for improving motivation and/or cognitive functions, in particular motivation and/or reference memory, in human individuals, who do not have defects of cognitive functions and/or reference memory deficits and/or motivation deficits, in particular for improving motivation and cognitive functions, in particular reference memory, in human individuals, who do not have reference memory deficits and motivation deficits.

18. Use according to claim 17, wherein
the cognitive function to be improved is the learning capability and/or the reference memory, in particular the learning capability and the reference memory.

19. Pharmaceutical preparation comprising at least one compound according to any one of claims 1 to 6 and a pharmaceutically acceptable carrier and/or diluent.

20. Pharmaceutical preparation according to claim 19 for use in therapy, in particular for treating age-related cognitive decline, more in particular for treating age-related cognitive decline according to 2018 ICD-10-CM Diagnosis Code R41.81.

21. Pharmaceutical preparation according to f claim 19 or 20 for use in improving motivation and/or cognitive functions in human individuals, in particular for use in improving motivation and the reference memory in human individuals, in particular for use in improving the reference memory and motivation in aging individuals.

22. Pharmaceutical preparation according to claim 19 for use in inhibiting DAT-mediated dopamine reuptake in the brain of a human individual.

23. Use of a compound according to any one of claims 1 to 6 for inhibiting DAT-mediated dopamine reuptake in the brain of, in particular healthy, human individuals who do not have defects of cognitive functions and/or reference memory deficits and/or motivation deficits.
